# EUROPEAN PATENT SPECIFICATION

(11) **EP 0 251 351 B1**
(45) Date of publication and mention of the grant of the patent: **11.08.1993**
(21) Application number: 87200863.6
(22) Date of filing: 08.05.1987
(51) Int. Cl.: B01J 21/10

(54) **Process for preparing stabilized magnesia and its use in catalytic processes**
Verfahren für die Herstellung von stabilisierter Magnesia und ihre Anwendung auf katalytische Verfahren
Procédé pour la préparation de magnésie stabilisée et son application dans des procédés catalytiques

(30) Priority: 23.05.1986 GB 8612687
(43) Date of publication of application: 07.01.1988
(73) Proprietor: SHELL INTERNATIONALE RESEARCH MAATSCHAPPIJ B.V., 2596 HR Den Haag (NL)
(72) Inventor: Schaper, Hennie, NL-1031 CM Amsterdam (NL); Kouwenhoven, Herman Wouter, NL-1031 CM Amsterdam (NL)

(56) References cited:
- EP-A- 0 183 225
- GB-A- 1 336 863
- GB-A- 1 336 864
- US-A- 2 361 613
- US-A- 3 791 992
- US-A- 4 269 735

## Description

The present invention relates to a process for the preparation of catalytically active compositions according to claim 1. Further the present invention relates to catalytically active compositions thus prepared as well as to their use in catalytic processes, e.g. in the isomerization of olefins.

Conventionally, silica, alumina or mixtures thereof are used as inert carriers for supported catalysts. Since these materials have acidic properties, they are less suitable for use as support or as catalysts in base-catalyzed processes. In addition, they are less attractive in processes wherein the acidic character of the catalysts or the catalysts supports may cause undesired side reactions. Therefore, interest has increased in the possible application of non acidic materials as catalysts or catalyst supports. In general, catalysts or catalyst supports should also have large surface areas and should be able to stand the reaction conditions of the processes wherein they will be applied. A material with a strong basic character is magnesia which therefore might be qualified as a suitable alternative. There are some problems, however, connected with the application of magnesia as a catalyst or a catalyst support.

Firstly, it appears to be very difficult to produce magnesia with a sufficiently high surface area and secondly, having obtained the appropriate high surface area magnesia, it appears to be very vulnerable when exposed to water or steam which causes an unacceptable loss of surface area.

Without wishing to be bound to any particular theory, it would appear that the surface area development of magnesia during calcination of its precursor, magnesium hydroxide, is caused by the breaking up of magnesium hydroxide crystallites into many small magnesium oxide crystallites. Water or steam, however, (mostly produced during the calcination), cause sintering of said small magnesium oxide crystallites, which effect is accompanied with an unacceptable loss of surface area. Therefore there is a strong incentive to investigate the possibility of preparing magnesia with a high surface area which is substantially maintained when exposed to water or steam.

In Journal of Catalysis 94, 547-557 (1985) materials with a relatively high surface area are described comprising magnesia and an oxide of a trivalent metal, such as aluminium. The molar ratio of magnesium and the trivalent metal ranges from 3-6 and a separate magnesium-containing phase is reported to be present in the materials formed at a Mg/Al³⁺ ratio above 3. As a consequence, however, the basicity of such modified magnesia is significantly reduced as exemplified by its unability to adequately catalyse the double-bond isomerisation of olefins, which is often used as a standard test to measure the degree of basicity. It seems that the presence of separate crystalline phases effectively reduces its catalytic activities for base catalysed reactions. It is also observed in said article that the presence of "non vaporizable" anions in the materials, i.e. anions which cannot be decomposed to yield hydroxyl ions, renders such materials substantially inactive for catalytic purposes which severely limits the choice in the starting materials.

Therefore, the present invention relates to a process for the preparation of catalytically active compositions according to claim 1. The molar Mg/Al ratio of the compositions according to the invention is preferably in the range from 5-10.

The compositions according to the invention can be characterized inter alia, by surface area determination, X-ray diffraction and IR-spectroscopy.

Surface area measurements indicated that the surface area of the compositions according to the invention is remarkably high (in the order of 200-250 m²/g) and the compositions appear moreover sufficiently stable, even in a steam-containing atmosphere. X-ray diffraction measurements on compositions according to the invention, before they have been calcined, show magnesium/aluminium hydroxy carbonate and no evidence of a separate magnesia phase. X-ray diffraction measurements and aluminium solid state NMR on the compositions according to the invention, after they have been calcined indicate also the absence of a separate magnesia phase. The IR measurements on compositions according to the invention indicate the absence of acidic sites which means that a trivalent metal moiety present in a homogeneous phase with said magnesia does not significantly disturb the basic properties of the said magnesium oxide.

Compositions according to the invention can potentially be applied as catalysts or catalyst supports, for instance in hydrogenation reactions or alkene oxidations. Due to their basic properties the compositions appear to be of special interest for application as catalysts or catalyst supports for base-catalyzed reactions such as polymerization of propylene oxide, double-bond isomerisation of olefins such as 1-pentene and aldol condensations.

Compositions according to the present invention may also be applied as components in catalytic cracking catalysts used in the catalytic cracking of hydrocarbon oils. They can be of advantage since the presence of basic materials such as magnesia may well cause a decrease in the extent of metal poisoning of the zeolitic catalysts normally applied in fluidized catalytic cracking processes. Stabilized magnesia is also expected to be of interest since its higher surface area is an intrinsic advantage for processes operated with frequent regeneration. The compositions according to the present invention may be present in the catalysts to be used but they can also be added to the feed.

Although compositions comprising a bulk molar Mg/Al ratio above 5 have been referred to in the Journal of Catalysis mentioned hereinbefore it should be noted that a seperate magnesium-containing phase is reported to be present in the materials formed.

It has now been found that compositions comprising magnesia and alumina in a homogeneous phase wherein the Mg/Al lent-metal molar ratio is at least 5 can be obtained by calcination of a precursor which has been prepared under controlled conditions, starting fro a magnesium salt and an aluminium salt. The invention therefore relates to a method for the preparation of catalytically active compositions comprising in a substantially homogeneous phase magnesia and alumina wherein the Mg/Al ratio is at least 5, which comprises mixing a solution comprising a magnesium salt and a salt of aluminium with a basic aqueous solution under the conditions according to claim 1, removing the reaction product from said reaction mixture, washing and drying said product and calcining the dried product at elevated temperature.

The properties of the ultimate reaction products appear to be strongly dependent on the conditions at which they have been prepared. It was experienced that an ultimate product comprising magnesia and alumina in a substantial homogeneous phase, can be obtained by mixing the solution comprising the metal salts and the basic solution at a substantially constant pH in the range from 7-10 and preferably at a pH of about 9. Furthermore, it has been found that mixing the above-mentioned components is best carried out at a substantially constant temperature in the range from 20-100 °C and preferably at a temperature of about 30-70 °C.

Examples of suitable salts comprise magnesium chloride and aluminium sulphate.

In order to obtain an ultimate product comprising in a substantially homogeneous phase magnesia and alumina wherein the Mg/Al metal molar ratio is at least 5, it will be clear that the Mg/Al metal molar ratio of the mixture comprising a magnesium- and an aluminium salt has to be also at least 5. The preparation is preferably carried out using a mixture of a magnesium salt and an aluminium salt wherein the Mg/Al ratio is ranging from 5-10.

In the preparation method according to the invention the best results are obtained when use is made of an aqueous carbonate solution.

The substantially solid reaction product may be removed batchwise or continuously from the reaction mixture and is dried, preferably at a temperature between 60 and 90 °C. In order to obtain catalytically active modified magnesia having a high surface area, the dried reaction product has to be calcined at elevated temperature. It was experienced that the calcination could well be carried out in air without inflicting any serious damage upon the magnesium oxide cristallytes newly formed during said calcination. The calcination is preferably carried out at a temperature ranging from 300-700 °C and more in particular at a temperature in the range from 400-600 °C.

The invention will now be illustrated by the following Examples.

### Example 1

a) A composition A was prepared as follows:
   An aqueous solution of 427.6 g = Mg(NO₃)₂.6H₂O and 111.1 g Al₂(SO₄)₃ 18H₂O (molar ratio Mg/Al=5) in a total volume of 2 litres (solution 1) is mixed thoroughly with 1 M aqueous solution of potassium hydroxide (solution 2) in a reaction chamber equipped with pH and temperature control. The feed rate of solution 1 is 2 l/h. The feed rate of solution 2 is continuously adjusted to maintain the pH of the mixture at a level of 9.0. The temperature is kept constant at 70 °C. The precipitate produced is continuously removed, filtered and washed with water. The washed precipitate is dried overnight at 80 °C and calcined at 400 °C for 18 hours.
   After said calcination the composition obtained (A) had a surface area of 250 m²/g. X-ray diffraction and IR measurements on a sample of said composition revealed a distorted magnesia pattern and the absence of acidic sites.
b) A composition B was prepared as described hereinabove with the difference that use was made of a solution containing both potassium hydroxide and potassium carbonate in a 3/1 molar ratio and the final calcination was carried out at 500 °C. The properties of the composition B are as described for composition A.
c) A further composition C was prepared by the method as described in b) above using the metal salts in such a ratio that the Mg/Al molar ratio in the homogeneous composition obtained amounted to 10. The composition was obtained after calcination at 600 °C.
d) The experiment as described in c) above was repeated whilst keeping the temperature of the mixture during the pH adjustment at 40 °C. The composition obtained after washing, drying and calcining had a homogeneity of 95%.

### Example 2

To investigate its steam stability composition A (obtained as described in Example 1) was heated at temperatures of 400-600 °C in a 50/50 steam/nitrogen flow (1 atm) for 20 hours.

For comparison high surface area magnesia (200 m²/g, obtained from magnesium hydroxide by calcination in vacuo) was exposed to the same treatment with steam.

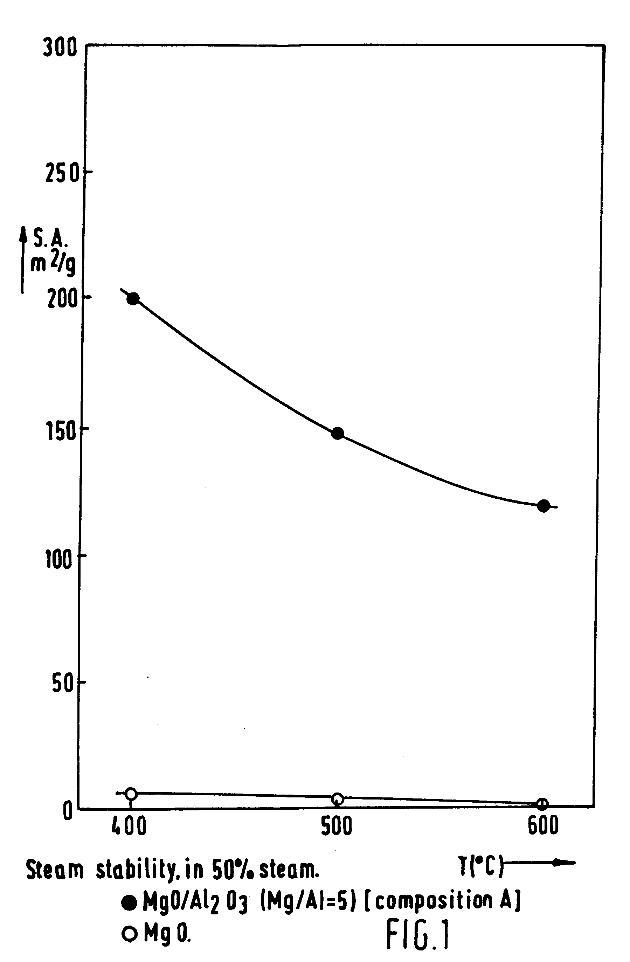

The results of the steam stability experiments are shown in Figure 1. The surface area in m²/g (plotted along the vertical axis) is expressed as a function of the temperature of the steam treatment in °C (plotted along the horizontal axis).

At 400 °C the steam stability of composition A (indicated by full circles) is very good and at higher temperatures only a relatively slight decrease in surface area occurs.

This is quite in contrast with conventional unmodified magnesia, which shows a very large decrease in surface area (indicated by open circles) even already at 400 °C.

### Example 3

a) Composition A in accordance with the invention was applied as a catalyst for the double-bond isomerisation of 1-pentene. The isomerisation was carried out in a tubular reactor (30 cm³) comprising a bed of composition A diluted with catalytically-inactive siliciumcarbide. 1-Pentene was passed through the catalyst bed at a temperature of 300 °C and with a space velocity of 0.7-1.2 l/h and was isomerised for 40-50% into cis and trans 2-pentene.
b) A similar experiment as described in a) above was carried out using composition B. Under the same conditions as applied when testing composition A no less than 86% of the starting material 1-pentene was isomerised into cis and trans 2-pentene.
c) A similar experiment was carried out using composition C. Under the same conditions as applied when testing composition A 50% of the starting material 1-pentene was isomerised into cis and trans 2-pentene.

Examples 1, 2 and 3 show that incorporation of a small amount of alumina into magnesia under formation of a homogeneous crystalline phase results in a high surface area material with a satisfactory steam stability even at temperatures well above 400 °C and a pronounced basic character which allows substantial isomerisation of 1-pentene.

## Claims

1. A method for preparing catalytically active compositions comprising in a substantially homogeneous crystalline phase magnesia and aluminium oxide, wherein the Mg/Al molar ratio is at least 5, which comprises mixing a solution comprising a magnesium salt and a salt of aluminium with a basic aqueous solution, comprising a carbonate ion, at a substantially constant pH ranging from 7-10, removing the reaction product from said reaction mixture, washing and drying said product and calcining the dried product at elevated temperature.

2. Method according to claim 4 whereby mixing is carried out at a pH of about 9.

3. Method according to claim 4 or 5 wherein the mixing is carried out at a substantially constant temperature ranging from 20-100 °C and in particular at a temperature of in the range from 30-70 °C.

4. Method according to any one of claims 4-6 wherein use is made of a Mg/trivalent-metal ratio in the range from 5-10.

5. Method according to any one of claims 4-7 wherein the reaction product obtained is dried at a temperature ranging from 60-90 °C.

6. Method according to any one of claims 4-8 wherein the dried product is calcined at a temperature ranging from 300-700 °C, in particular at a temperature in the range 400-600 °C.

7. Catalytically active compositions prepared according to any one of claims 1-6.

8. Process for the isomerization of olefins wherein use is made of catalytically active compositions according to claim 7.

9. Process for the catalytic cracking of hydrocarbon wherein use is made of catalytically active compositions according to claim 7.

## Patentansprüche

1. Ein Verfahren zur Herstellung katalytisch aktiver Zusammensetzungen, umfassend Magnesiumoxid und Aluminiumoxid in einer im wesentlichen homogenen kristallinen Phase, wobei das Mg/Al-Molverhältnis mindestens 5 beträgt, welches Verfahren das Mischen einer Lösung, die ein Magnesiumsalz und ein Salz von Aluminium enthält, mit einer basischen wäßrigen Lösung, die ein Carbonation enthält, bei einem im wesentlichen konstanten pH-Wert von 7 bis 10, das Entfernen des Reaktionsprodukts aus der genannten Reaktionsmischung, das Waschen und Trocknen des genannten Produkts und das Calcinieren des getrockneten Produkts bei erhöhter Temperatur umfaßt.

2. Verfahren nach Anspruch 1, wobei das Mischen bei einem pH-Wert von ca. 9 stattfindet.

3. Verfahren nach Anspruch 1 oder 2, wobei das Mischen bei im wesentlichen konstanter Temperatur im Bereich von 20 bis 100°C und vor allem im Bereich von 30 bis 70°C stattfindet.

4. Verfahren nach einem der Ansprüche 1 bis 3, in welchem das Verhältnis von Mg zu dreiwertigem Metall im Bereich von 5 bis 10 liegt.

5. Verfahren nach einem der Ansprüche 1 bis 4, wobei das erhaltene Reaktionsprodukt bei einer Temperatur im Bereich von 60 bis 90°C getrocknet wird.

6. Verfahren nach einem der Ansprüche 1 bis 5, wobei das getrocknete Produkt bei einer Temperatur im Bereich von 300 bis 700°C , vor allem bei einer Temperatur im Bereich von 400 bis 600°C calciniert wird.

7. Katalytisch aktive Zusammensetzungen, hergestellt nach einem der Ansprüche 1 bis 6.

8. Verfahren zum Isomerisieren von Olefinen, bei welchem katalytisch aktive Zusammensetzungen nach Anspruch 7 eingesetzt werden.

9. Verfahren zum katalytischen Cracken von Kohlenwasserstoff, in welchem katalytisch aktive Zusammensetzungen nach Anspruch 7 eingesetzt werden.

## Revendications

1. Un procédé pour préparer des compositions actives du point de vue catalytique, comportant dans une phase cristalline pratiquement homogène de la magnésie et de l'oxyde d'aluminium, le rapport molaire Mg/Al étant d'au moins 5, qui comporte le mélange d'une solution d'un sel de magnésium et d'un sel d'aluminium avec une solution aqueuse basique comportant un ion carbonate à un pH pratiquement constant se situant dans la gamme de 7 à 10 ; l'élimination du produit de réaction à partir dudit mélange réactionnel, le lavage et le séchage du produit et la calcination du produit séché à température élevée.

2. Procédé selon la revendication 1, dans lequel le mélange est mis en oeuvre à un pH d'environ 9.

3. Procédé selon la revendication 1 ou 2, dans lequel le mélange est mis en oeuvre à une température pratiquement constante dans la gamme de 20 à 100°C et en particulier à une température dans la gamme de 30 à 70°C.

4. Procédé selon l'une quelconque des revendications 1 à 3, dans lequel on utilise un rapport Mg/métal trivalent dans la gamme de 5 à 10.

5. Procédé selon l'une quelconque des revendications 1 à 4, dans lequel le produit de réaction obtenu est séché à une température dans la gamme de 60 à 90°C.

6. Procédé selon l'une quelconque des revendications 1 à 5, selon lequel le produit séché est calciné à une température dans la gamme de 300 à 700°C, en particulier à une température dans la gamme de 400 à 600°C.

7. Compositions actives du point de vue catalytique, préparées selon l'une quelconque des revendications 1 à 6.

8. Procédé pour l'isomérisation des oléfines, dans lequel on utilise des compositions actives du point de vue catalytique selon la revendication 7.

9. Procédé pour le craquage catalytique des hydrocarbures dans lequel on utilise des compositions actives du point de vue catalytique selon la revendication 7.
